# EUROPÄISCHE PATENTSCHRIFT

(11) **EP 3 047 267 B1**
(45) Veröffentlichungstag und Bekanntmachung des Hinweises auf die Patenterteilung: **08.01.2020**
(21) Anmeldenummer: 14766913.9
(22) Anmeldetag: 17.09.2014
(51) Int. Cl.: G01N 33/15, B01D 61/28, G01N 13/00

(54) **DIALYSEZELLE FÜR EINE IN-VITRO-FREISETZUNGSTESTAPPARATUR, VERWENDUNG DER DIALYSEZELLE UND IN-VITRO-FREISETZUNGSTESTAPPARATUR**
DIALYSIS CELL FOR AN IN-VITRO RELEASE TEST APPARATUS, USE OF THE DIALYSIS CELL AND IN-VITRO RELEASE TEST APPARATUS
CELLULE DE DIALYSE POUR UN APPAREIL DE TEST DE LIBÉRATION IN VITRO, UTILISATION DE LA CELLULE DE DIALYSE ET APPAREIL DE TEST DE LIBÉRATION IN VITRO

(30) Priorität: 18.09.2013 DE 102013015522
(43) Veröffentlichungstag der Anmeldung: 27.07.2016
(73) Patentinhaber: Pharma Test Apparatebau AG, 63512 Heinburg (DE)
(72) Erfinder: WACKER, Matthias, 60389 Frankfurt/Main (DE); JANAS, Christine, 61440 Oberursel (DE)
(74) Vertreter: Castell, Klaus
(86) Internationale Anmeldenummer: PCT/EP2014/002523
(87) Internationale Veröffentlichungsnummer: WO 2015/039749

(56) Entgegenhaltungen:
- JP-A- H01 187 453
- US-A- 2 692 854
- US-A1- 2007 205 155
- US-A1- 2010 221 838
- MONA M.A. ABDEL-MOTTALEB ET AL: "Standardized in vitro drug release test for colloidal drug carriers using modified USP dissolution apparatus I", DRUG DEVELOPMENT AND INDUSTRIAL PHARMACY, Bd. 37, Nr. 2, Februar 2011 (2011-02), Seiten 178-184, XP055155043, ISSN: 0363-9045, DOI: 10.3109/03639045.2010.502534
- YUAN GAO ET AL: "In Vitro Release Kinetics of Antituberculosis Drugs from Nanoparticles Assessed Using a Modified Dissolution Apparatus", BIOMED RESEARCH INTERNATIONAL, Bd. 2013, 136590, 10. Juli 2013 (2013-07-10), Seiten 1-9, XP055155050, ISSN: 2314-6133, DOI: 10.1155/2013/136590
- "[711] DISSOLUTION", Stage 6 Harmonization, 1. Dezember 2011 (2011-12-01), Seiten 1-8, XP055155057, Gefunden im Internet: URL:http://www.usp.org/sites/default/files /usp_pdf/EN/USPNF/2011-02-25711DISSOLUTION .pdf [gefunden am 2014-11-25]

## Beschreibung

Die Erfindung betrifft eine Dialysezelle für eine In-vitro-Freisetzungstestapparatur. Die Invitro-Freisetzungstestapparatur dient dazu, das Freisetzungsverhalten, wie die Freisetzungsrate, einer Substanz einer Stoffzusammensetzung, wie eines Arzneimittels, welche Substanz an Molekularträger, wie Liposome, Mizellen oder Nanopartikel, gebunden ist, zu ermitteln.

In-vitro-Freisetzungstestapparaturen sind beispielsweise bekannt aus dem Europäischen Arzneibuch (Ph. Eur.) und der US Pharmacopoeia (USP), etwa die Apparatur 2 (Blattrührer-Apparatur) des Europäischen Arzneibuchs, die USP-Apparatur II oder vergleichbare, zweckähnliche Apparaturen insbesondere weiterer Arzneibücher.

Die Funktionsweise einer derartigen In-vitro-Freisetzungstestapparatur wird in der Fachliteratur beschrieben, wobei beispielhaft auf die Publikation in dem International Journal of Pharmaceutics 159 (1997), Seiten 27-33, "Method for evaluating drug release from liposomes in sink conditions" verwiesen wird.

Bei der bekannten In-vitro-Freisetzungstestapparatur wird ein Dialysebeutel, in dem die Stoffzusammensetzung eingebracht ist, in einen Aufnahmebehälter eingebracht, in dem der Dialysebeutel frei in einer Substanzaufnahmeflüssigkeit schwimmen kann. Der Dialysebeutel besteht aus einer Dialysemembran, der die gebundenen Substanzen der Stoffzusammensetzung innerhalb des Beutels zurückhält, wobei die Dialysemembran das Substanzaufhahmefluid sowie den in das Substanzaufnahmefluid freigesetzte Substanz durchlässig ist. Um aus einer schlauchförmigen Dialysemembran einen Beutel zu bilden, werden die Enden der schlauchförmigen Dialysemembran beispielsweise mittels Klammern verschlossen.

Es zeigte sich, dass mit der bekannten In-vitro-Freisetzungstestapparatur, bei der ein Dialysebeutel frei in der Substanzaufnahmeflüssigkeit schwimmt, auch dann keine ausreichend reproduzierbaren Messergebnisse erzielt werden können, wenn eine Rühreinheit dafür sorgt, die Dialysemembran ständig in Bewegung innerhalb der Substanzaufnahmeflüssigkeit zu halten. Die mögliche Austauschfläche des Dialysebeutels kann aufgrund des kaum vereinheitlichbaren Verschließvorgangs mit Klammern an den Schlauchenden kaum nachgearbeitet werden. Diese Verschließtechnik lässt keine konstanten Austauschflächen zu, jedoch eine derart starke Variation, dass sich das bekannte In-vitro-Freisetzungstestverfahren nicht für ein Standardverfahren eignet. Auch das Umherschwimmen des Dialysebeutels in einem das Substanzaufnahmefluid haltenden Behälter lässt kaum ein vorbestimmbares Testverfahren zu, da der Dialysebeutel beliebig in Kontakt mit der Behälterwand gelangen kann. Insofern kann eine über längeren Zeitraum konstante, reproduzierbare Austauschfläche an der Dialysemembran des Dialysebeutels nicht sichergestellt werden.

Weitere Dialysezellen sind etwa aus der US 2010/221838 A1 und JP H01 187453 A bekannt, welche jeweils eine austauschbare Dialysezelle mit Haltegerüst offenbaren. Die US 2692854 A offenbart weiterhin eine Dialysezelle mit Innenrührer.

Es ist Aufgabe der Erfindung, die Nachteile des Stands der Technik zu überwinden, insbesondere eine In-vitro-Freisetzungstestapparatur zu schaffen, mit der eine standardisierbare, wiederholbare Ermittlung des Substanzfreisetzungsverhaltens durchgeführt werden kann.

Diese Aufgabe wird durch die Merkmale von Anspruch 1 gelöst.

Danach ist eine Dialysezelle für eine In-vitro-Freisetzungstestapparatur vorgesehen. Die Dialysezelle ermöglicht das Ermitteln eines Freisetzungsverhaltens, wie einer Freisetzungsrate, einer Substanz einer Stoffzusammensetzung, wie eines Arzneimittels, welche Substanz an Molekularträgern, wie Liposome oder Nanopartikel, gebunden ist, gegenüber einem Substanzaufnahmefluid. Eine Substanz kann beispielsweise ein Wirkstoff oder Zusatzstoff einer Stoffzusammensetzung in Form eines Arzneimittels sein oder ein Lebensmittelzusatzstoff, Hilfsstoff, oder dergleichen einer Stoffzusammensetzung in Form eines Lebensmittels. Das Freisetzungsverhalten soll in-vitro, also außerhalb eines lebenden Organismus, beispielsweise in einem Reagenzglas ermittelt werden. Dafür wird als Substanzaufnahmefluid oder Umgebungsfluid ein Körpersimulationsfluid verwendet, das ein Organismusfluid simulieren soll. Das Substanzaufnahmefluid kann beispielsweise Magensaft, Darmsaft oder Blut sein. Als Substanzaufnahmefluid kann auch eine Pufferlösung, eine Prüfflüssigkeit, beispielsweise gemäß Kapitel 5.17.1 der 7. Ausgabe des Europäischen Arzneibuchs oder dergleichen verwendet werden. Die Molekularträger, welche die Substanz tragen sollen, können Mikrooder Nanopartikel, wie Liposome, Lipidkomplexe, Mizellen, synthetische Polymere, natürliche Polymere oder anorganische Verbindungen, sein. Die Molekularträger haben Oberflächen, an denen die Substanz gebunden sein soll. Es können auch Molekularträger verwendet werden, die in deren Inneren zumindest eine Substanz gebunden halten. Durch die Testung mittels der In-vitro-Freisetzungstestapparatur soll herausgefunden werden, wie oder in welcher Geschwindigkeit die Substanz, wie die Arznei, welche beispielsweise in Form einer Suspensionen oder Emulsionen verabreicht wird, in den Organismus übergehen kann.

Die erfindungsgemäße Dialysezelle umfasst einen abgeschlossenen Zelleninnenraum. Der Zelleninnenraum ist zum Aufnehmen der Stoffzusammensetzung und des Substanzaufnahmefluids vorgesehen. Des Weiteren hat die Dialysezelle eine den Zellinnenraum zumindest teilweise begrenzende Dialysemembran, die die gebundene Substanz zurückhält, also an einem Verlassen der Dialysezelle oder am Eindringen in die Dialysezelle hindert. Ein Durchlass des Substanzaufnahmefluids durch die Dialysemembran ist im Wesentlichen gewährleistet. Das Substanzaufnahmefluid kann zu einem geringen Teil Moleküle umfassen, für die die Dialysemembran nicht passierbar ist. Des Weiteren ist die Dialysemembran auch für die in das Substanzaufnahmefluid freigesetzte Substanz durchlässig. Erfindungsgemäß ist die Dialysemembran an einer Tragstruktur befestigt, die das Membranmaterial in einer festgelegten und stabilen Gestalt hält. Mittels der Tragstruktur kann die Dialysezelle an einer gegenüber der In-vitro-Freisetzungstestapparatur ortsfesten Montageposition fest angebracht werden. Die Tragstruktur kann wie ein Rahmen fungieren, an dem vorzugsweise die flexible Dialysemembran aufgespannt ist. Damit ist die Austauschoberfläche der Dialysemembran für die freigesetzte Substanz während des gesamten Testverfahrens festgelegt und konstant, was ein reproduzierbares Verfahren bewirkt und eine Standardisierung ermöglicht. Durch die ortsfeste Anbringung der Dialysezelle insbesondere im Hinblick auf weitere Bauteile der In-vitro-Freisetzungstestapparatur, wie ein Aufnahmebehälter, beispielsweise ein sogenanntes "Vessel" etwa gemäß der US Pharmacopoeia (USP) oder dem Europäischen Arzneibuch (Ph. Eur.) oder ein Reagenzglas, wird eine unerwünschte Kontaktaufnahme der Austauschfläche der Dialysemembran an dem jeweiligen Apparaturbauteil während des gesamten Testverfahrens verhindert. Versuche zeigten, dass beim Einsatz der erfindungsgemäßen Dialyse in der In-vitro-Freisetzungstestapparatur bei identischer Stoffzusammensetzung und Substanzmenge übereinstimmende Freisetzungsdaten ermittelt werden konnten, was es ermöglicht, einen Vergleich zwischen verschiedenen Stoffzusammensetzungen, was deren Freisetzungsverhalten betrifft, vorzunehmen.

Nach der Erfindung hat die Tragstruktur ein Haltegerüst, wie einen Käfig, und zumindest eine apparaturseitiges, aufnahmebehälterseitiges Montageteil, das als Montageplatte mit einem Außenringflansch ausgebildet ist. An dem Außenringflansch ist das Montageteil, ergo die Tragstruktur, an die Invitro-Freisetzungstestapparatur lösbar befestigt. Auf diese Weise kann die Dialysezelle gegen eine andere Dialysezelle ausgetauscht werden, ohne den grundsätzlichen Aufbau der In-vitro-Freisetzungstestapparatur ändern zu müssen. Die Position der Dialysezelle bleibt bei der Durchführung jedes einzelnen Tests immer die gleiche, wodurch identische Testbedingungen bereitgestellt werden. Zusätzlich ist das Montageteil gegenüber dem Haltegerüst als separates Bauteil derart an dem Haltegerüst lösbar befestigt, dass die Dialysemembran zwischen dem

Haltegerüst und dem Montageteil unter Ausbildung einer zuverlässigen Dichtfläche angeordnet, vorzugsweise eingeklemmt, ist. Auf diese Weise werden eine gegenüber einer Außenseite der Tragstruktur geschlossene umlaufende Dichtfläche und eine leicht realisierbare Dialysezellenform, wie eine zylindrische Dialysezellenform, gebildet.

Das Montageteil hat einen Durchgang für einen in Axialrichtung erstreckenden Antriebsstrang, wie eine Antriebswelle, zum Betätigen eines im Zellinnenraum anzuordnenden Innenrührer. Des Weiteren hat das Montageteil eine zylindrische Montagefläche, die vorzugsweise radial nach außen weist, und eine zylindrische Dichtfläche aufweisen, die vorzugsweise radial nach innen auf das Haltegerüst weist. Das Haltegerüst hat eine an die Dichtfläche formangepasste, radial nach außen gewandte, vorzugsweise zylindrische Dichtgegenfläche, wobei zwischen der Dichtfläche und der Dichtgegenfläche die Dialysemembran eingeklemmt ist. Es sei klar, dass statt einer Einklemmung zum Erreichen der Dichtflächen, auch eine Klebung oder andersartige dichtende Befestigungsmöglichkeiten herangezogen werden können
Bei einer bevorzugten Ausführung der Erfindung kann die Dialysemembran außenumfänglich an der Tragstruktur, insbesondere an dem Haltegerüst, abdichtend befestigt sein, beispielsweise mittels eines Klebers oder einer Verschweißung. Vorzugsweise ist zum Befestigen der Dialysemembran außenumfänglich an dem Haltegerüst der Tragstruktur wenigstens ein Haltering vorgesehen, der, wie ein Dichtungsring oder O-Ring, elastisch und/oder, wie ein Schrumpfschlauch, plastisch verformbar sein kann, um eine abdichtende Befestigung der Dialysemembran an dem Haltegerüst zu gewährleisten. Es sei klar, dass durch die elastische und/oder plastische Verformung des Halterings eine ausreichend große Haltekraft wie auch eine ausreichend große Verformung bereitgestellt sein muss, um Undichtigkeiten zwischen dem Haltegerüst und der Dialysemembran zu vermeiden. Vorzugsweise sind wenigstens zwei Halteringe an dem Haltegerüst angeordnet, jeweils einer oberhalb und einer unterhalb der Dialysekammer. Bevorzugt ist an der Tragstruktur, insbesondere dem Haltegerüst, wenigstens eine Halteringaufnahme, wie eine Ringnut, für den wenigstens einen Haltering vorgesehen beziehungsweise jeweils eine Halteringaufnahme für die mehreren Halteringe. Es ist klar, dass eine durch Halteringe befestigte Dialysemembran frei von einer Klemmbefestigung durch die Tragstruktur beziehungsweise deren Haltegerüst und dazu benachbarte Bauteile, wie ein Klemmteil oder ein Montageteil, sein kann. Bei einer bevorzugten Weiterbildung der Erfindung formt das Haltegerüst eine insbesondere käfigartige Laternen- oder Jochstruktur. Zusätzlich kann das Haltegerüst zwei sich gegenüber liegende, vorzugsweise plattenförmige Basisendabschnitte sowie die Basisendabschriitte koppelnde Säulen umfassen. Die Basisendabschnitte stellen die axialen Enden des Haltegerüsts dar, zwischen denen sich die Säulen insbesondere geradlinig axial erstrecken. Vorzugsweise verlaufen die Säulen insbesondere parallel zu einem sich in dem Zellinnenraum anzuordnenden Antriebsstrang, wie der Antriebswelle.

Bei einer bevorzugten Ausführung der Erfindung bilden die Säulen mit den Basisendabschnitten des Haltegerüsts einen insbesondere von der Dialysemembran radial geschlossenen Zellenfensterrahmen, der die Zellenfensteröffnungen begrenzt, welche durch die Dialysemembran verschlossen sind. Durch die Fensteröffnungen findet der Austausch der freigesetzten Substanz aus der Dialysezelle oder in die Dialysezelle statt. Vorzugsweise erfolgt ausschließlich über die durch den Fensterrahmen begrenzten Zellenfenster der Substanzdurchlass durch die Dialysemembran hindurch aus der oder in die Dialysezelle.

Bei einer bevorzugten Ausführung der Erfindung liegen die radialen Außenseiten der Basisendabschnitte sowie auch die radialen Außenseiten der Säulen in einer gemeinsamen zylindrischen Hüllebene, so dass die Dialysemembran insbesondere im Wesentlichen eigenspannungsarm an den Außenseiten flächig anliegt, so dass die an der Tragstruktur angebrachte Dialysemembran eine zylinderförmige Mantelstruktur bildet. Es sei klar, dass die Dialysemembran dann als Eigenspannungsarm anliegend betrachtet werden kann, wenn die Dialysemembran in Längsrichtung und in Umfangsrichtung wenigstens genauso groß ist wie die entsprechenden Längs- und Umfangsabmessungen der Tragstruktur, so dass auf eine an der Tragstruktur befestigte Dialysemembran keine Zugkräfte insbesondere aufgrund von Dehnung wirken. Vorzugsweise haben der apparaturnahe Basisendabschnitt des Haltegerüsts und das Montageteil eine fluchtende Einführöffnung, über den beispielsweise die Stoffzusammensetzung dem Zellinnenraum zuführbar, insbesondere nachfüllbar, ist. Vorzugsweise kann durch die Einführöffnung eine Probenentnahme aus dem Zelleninnenraum durchgeführt werden, anhand der beispielsweise eine Substanzkonzentration im Zelleninnenraum ermittelbar ist, so dass die Dialysezelle auch besonders gut für sogenannte "reversed-dialysis"-In-vitroFreisetzungstestverfahren geeignet ist, bei denen eine gebundene Substanz außerhalb des Zellinnenraums in die In-vitro-Freisetzungstestapparatur eingebracht wird, so dass die ungebundene Substanz durch die Dialysemembran in den Zelleninnenraum eindringen und die Konzentration bzw. Konzentrationsänderung der Substanz im Substanzaufnahmefluid im Zelleninnenraum ermittelt werden kann.

Bei einer Weiterbildung der Erfindung umfasst die Tragstruktur ein apparaturfernes, vorzugsweise plattenförmiges, Klemmteil zum Einklemmen der Dialysemembran an ein Haltegerüst der Tragstruktur. Das Klemmteil kann insbesondere eine vorzugsweise zylindrische Dichtfläche aufweisen, die vorzugsweise radial nach innen auf das Haltegerüst weist. Das Haltegerüst kann insbesondere eine an die Dichtfläche formangepasste, radial nach außen gewandte, zylindrische Dichtgegenfläche formen, wobei insbesondere zwischen der Dichtfläche und der Dichtgegenfläche die Dialysemembran eingeklemmt ist. Somit können auf beiden Ringendseiten der Dialysemembran insbesondere gleichartige umlaufende Dichtflächen gebildet sein. Das Klemmteil formt eine von dem Zellinnenraum abgewandte insbesondere plane Tragfläche, an der ein Außenrührer gehalten sein kann. Vorzugsweise ist die Tragstruktur mit einer Verdrehsicherung zum Vermeiden einer insbesondere rotatorischen Relativbewegung zwischen Klemmteil und Haltegerüst, also zwischen Dichtfläche und Dichtgegenfläche, ausgestattet. Eine Verdrehsicherung kann realisiert sein, indem, das Haltegerüst, insbesondere an der Dichtgegenfläche, eine vorzugsweise in Axialrichtung hervorstehende Nase aufweist und in das Klemmteil, insbesondere die Dichtfläche, eine vorzugsweise zumindest in Umfangsrichtung zu der Nase formkomplementäre Ausnehmung eingebracht ist. Es sei klar, dass die Verdrehsicherung gegenüber einer Achse der Tragstruktur und/oder des Montageteils exzentrisch angeordnet sein kann.

Bei einer Weiterbildung der Erfindung hat die Dialysemembran die Form eines flexiblen, insbesondere im montierten Zustand in dessen Längsrichtung konstant hohl-zylindrischen Schlauchs. Der flexible Schlauch kann im montierten Zustand jegliche Hohlform bilden. Vor zugsweise ist der lichte Querschnitt der Hohlform in axialer Längsrichtung des Schlauchs konstant. Die Dialysemembran kann die Tragstruktur derart mantelförmig umgeben, dass durch die Dialysemembran gebildete Zellinnenraumgrenzwände sich parallel zu einer Längsachse der Tragstruktur, insbesondere zu einem in den Zellinnenraum ragenden Antriebsstrang, wie einer Antriebswelle, zum Betätigen eines im Zelleninnenraum anzuordnenden Innenrührers, erstrecken.

Bei einer Weiterbildung der Erfindung ist im Zellinnenraum ein Innenrührer mit einem im Zellinnenraum vollständig liegenden Innenrührorgan, wie einem Rührexzenter angeordnet. Das Innenrührorgan kann beispielsweise als magnetischer Schwimmer innerhalb der Dialysezelle ausgebildet sein. Vorzugsweise ist der Innenrührer mit einer mit dem Innenrührorgan direkt gekoppelten Antriebswelle versehen, die sich von dem Innenrührorgan aus der Dialysezelle heraus dichtend erstreckt, um betägigbar zu sein. Vorzugsweise ist das Innenrührorgan dauermagnetisch, um andere beispielsweise externe Rührorgane mitanzutreiben. Bei einer bevorzugten Ausführung ist das Innenrührorgan ein Steckteil zum Halten eines Dauermagneten, insbesondere eines Dauermagnetenpaares. Das Steckteil kann am Ende der Antriebswelle vorzugsweise abnehmbar aufgesteckt werden. Vorzugsweise erstreckt sich die direkte Antriebswelle für das Innenrührorgan durch eine Lageröffnung sowohl in dem Basisendabschnitt der Tragstruktur als auch in dem Montageteil der Tragstruktur hindurch. Außerdem kann eine Dichtung zwischen der Antriebswelle und der Tragstruktur vorgesehen sein.

Bei einer Weiterbildung der Erfindung ist an einem apparaturfernen Abschnitt der Tragstruktur außenseitig ein Außenrührer angeordnet, der mit einem in den Zellinnenraum angeordneten Innenrührer derart insbesondere berührungslos gekoppelt ist, dass ein direkter Antrieb für einen Rührer, insbesondere für den Innenrührer, auch den anderen Rührer, insbesondere den Außenrührer, antreibend betätigt. Ein Außenrührorgan des Außenrührers kann dauermagnetisch sein oder zumindest derart ferromagnetisch reagieren, dass das Außenrührorgan unter dem Einfluss magnetischer Anziehungskräfte einem getriebenen Innenrührorgan des Innenrührers folgt und/oder an der Tragstruktur, insbesondere dem Klemmteil, gehalten wird. Auf diese Weise ist nur ein einziger Antrieb für zwei Rührer, insbesondere einen Innen- und ein Außenrührer, notwendig. Bei einer alternativen Weiterbildung der Erfindung kann der Außenrührer insbesondere formschlüssig oder kraftschlüssig mittels einer Antriebswellenkoppelung an den Innenrührer bewegungsübertragungsgemäß gekoppelt sein, wobei sich zumindest ein Teil der Wellenkopplung vorzugsweise abdichtend durch eine Öffnung der Dialysezelle erstreckt.

Des Weiteren betrifft die Erfindung die Verwendung einer erfindungsgemäßen Dialysezelle für eine In-vitro-Freisetzungstestapparatur.

Schließlich betrifft die Erfindung eine In-vitro-Freisetzungstestapparatur zum Ermitteln des Freisetzungsverhaltens, wie der Freisetzungsrate, einer Substanz einer Stoffzusammensetzung, wie eines Arzneimittels, welche Substanz an Molekularträger, wie Liposome, Mizellen oder Nanopartikel, gebunden ist, gegenüber einem Substanzaufnahmefluid. Die In-vitro-Freisetzungstestapparatur umfasst eine erfindungsgemäße Dialysezelle und einen die Dialysezelle zumindest teilweise, vorzugsweise vollständig, umgebenden Umgebungsraum, zum Aufnehmen des Substanzaufnahmefluids. Bei dem "reversed-dialysis"-In-vitro-Freisetzungstestverfahren wird die Stoffzusammensetzung mit der Substanz direkt in den Umgebungsraum gegeben. Diese Substanzkonzentration kann grundsätzlich in der Dialysezelle und/oder in dem Umgebungsraum ermittelt werden, insbesondere durch Probenentnahme.

Bei einer Weiterbildung der Erfindung hat die In-vitro-Freisetzungstestapparatur einen Aufnahmebehälter zum Begrenzen des Umgebungsraums, von dessen Wandung, vorzugsweise dessen Deckel, sich ein Montagearm in das Behälterinnere erstreckt. Der Montagearm kann insbesondere einen endseitigen Montageflansch aufweisen, an dem die Tragstruktur der Dialysezelle befestigt ist. Der Montagearm kann lösbar an der Wandung befestigt sein. Vorzugsweise kann der Montagearm eine insbesondere käfigförmige Laternen- oder Jochstruktur formen. Dabei kann der Montagearm zwei sich axial gegenüber liegende, vorzugsweise plattenförmige Grundendabschnitte sowie die Grundendabschnitte koppelnde Stützen umfassen, die sich vorzugsweise parallel zu einem in dem Behälterinnenraum anzuordnen Antriebsstrang erstrecken. Der Antriebsstrang, vorzugsweise die Antriebswelle, kann sich für einen in den Zellinnenraum angeordneten Innenrührer durch die Wandung erstrecken, wobei insbesondere der Antriebsstrang an der Wandung drehgelagert sein kann. Vorzugsweise ist der Antriebsstrang zweigeteilt, wobei sich ein zellenseitiges Strangteil von dem Zellinnenraum in den Aufnahmebehälter erstrecken kann, wobei sich ein zellfernes Strangteil von dem Behälterinneren zur Außenseite des Behälters erstrecken kann. Die Grundendabschnitte können auch durch ein zellenseitiges Strangteil als Mittelstütze gekoppelt sein. Vorzugsweise weist der Deckel einen Deckeleinsatz zum Bereitstellen einer Zugriffsmöglichkeit auf die Dialysezelle auf, wobei der Deckel und gegebenenfalls der Deckeleinsatz wenigstens einen Dichtkörper zum abdichtenden Verschließen des Aufnahmebehälters aufweisen kann.

Mit dem Erfindungsgegenstand ist es möglich, ein standardisierbares In-vitro-Freisetzungsverfahren zu schaffen. Zudem kann eine bestehende In-vitro-Freisetzungstestapparatur ohne weiteres mit der erfindungsgemäßen Dialysezelle upgegradet werden, ohne bauliche Veränderungen an der bestehenden Apparatur vornehmen zu müssen. Selbst die Dialysezelle muss nach einem durchgeführten Test nicht vollständig entsorgt werden, lediglich die Dialysemembran oder zumindest muss diese gereinigt werden, wobei das Traggerüst der Dialysezelle sowie die Montageteile mehrmals verwendet werden können.

Weitere Eigenschaften, Vorteile und Merkmale der Erfindung werden durch die folgende Beschreibung einer bevorzugten Ausführung anhand der beiliegenden Zeichnungen deutlich, in denen zeigen:
- Figur 1: eine Querschnittsansicht einer erfindungsgemäßen In-vitro-Freisetzungstestapparatur, die eine erfindungsgemäße Dialysezelle umfasst;
- Figur 2: eine perspektivische Ansicht eines Behälterinneren in einer In-vitro-Freisetzungstestapparatur angeordneten Montagearms zum Halten der erfindungsgemäßen Dialysezelle;
- Figur 3: eine perspektivische Ansicht einer käfigförmigen Tragstruktur der erfindungsgemäßen Dialysezelle;
- Figur 4: eine perspektivische Ansicht eines apparaturfernen Klemmteils zum Fixieren einer Dialysemembran der erfindungsgemäßen Dialysezelle; und
- Figur 5: eine perspektivische Ansicht eines apparaturseitigen Montageteils der Tragstruktur einer erfindungsgemäßen Dialysezelle.

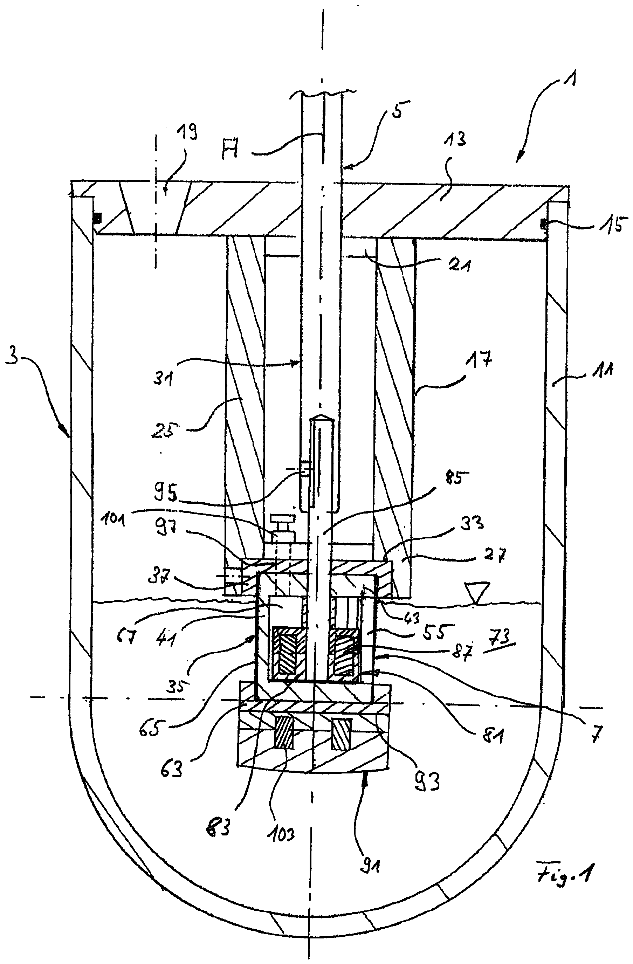

In Figur 1 ist die erfindungsgemäße In-vitro-Freisetzungstestapparatur im Allgemeinen mit der Bezugsziffer 1 versehen, die drei Hauptbestandteile umfasst, nämlich einen Aufnahmebehälter 3, eine Antriebseinrichtung 5 sowie eine erfindungsgemäße Dialysezelle 7, die im Inneren der Aufnahmebehälters 3 angeordnet und von der Antriebseinrichtung 5 betätigt ist.

Der Aufnahmebehälter 3 besteht aus einem Gefäß 11, wie einem Reagenzglas und einem Deckel 13, der eine obere Öffnung des Gefäßes 11 mittels eines O-Rings 15 abdichtet.

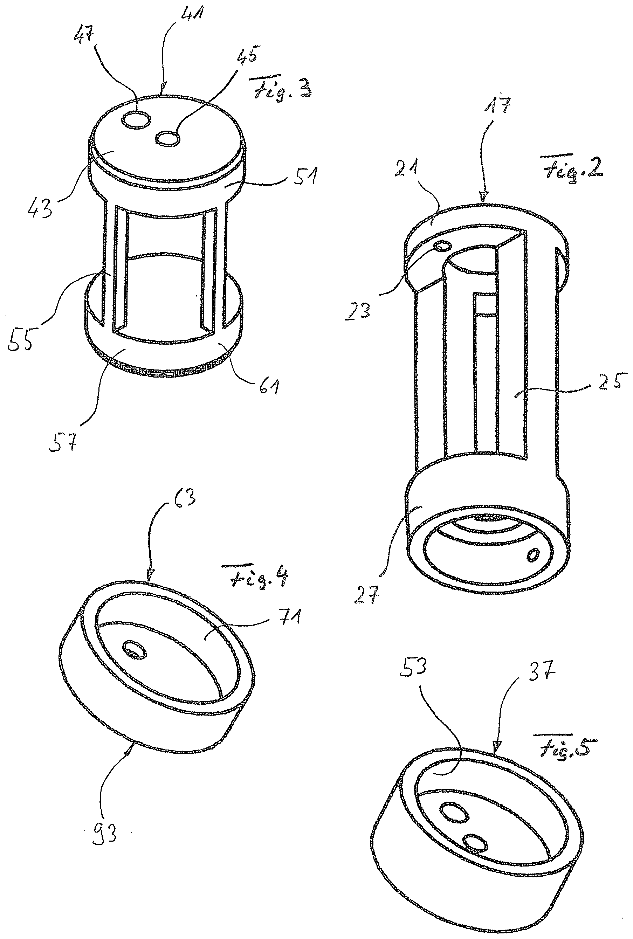

Von der Innenseite des Deckels 13 ragt ein Montagearm 17 in das Innere des Gefäßes 11. Der Montagearm 17 ist näher in Figur 2 dargestellt. Der Montagearm 17 umfasst einen deckelseitigen, plattenringförmigen Grundendabschnitt 21, der über Bohrungen 23 an die Innenseite des Deckels 13 lösbar verschraubt ist. Von dem deckelseitigen Grundendabschnitt 21 erstreckt sich ein Paar Stützen 25 parallel zur Axialrichtung A und der Antriebseinrichtung 5 und mündet in einem dialysezellenseitigen Grundendabschnitt 27. Der gesamte Montagearm 17 umfasst einen axialen Durchgangskanal zur Durchführung eines Antriebstrangs 31 der Antriebseinrichtung 5. Der dialysezellenseitige Grundendabschnitt 27 umfasst einen radial nach innen gewandten Absatz 33, in dem eine Teil der Tragstruktur 35 der Dialysezelle 7 aufgenommen ist, nämlich das ringplattenförmige Montageteil 37. Das ringförmige Montageteil 37 ist im Querschnitt U-förmig, wobei die kurzen U-Schenkel mit dem Grundendabschnitt 27 des Montagearms 17 gekoppelt sind.

Die Tragstruktur 35 der Dialysezelle hat außerdem ein Traggerüst 41, das näher in Figur 3 gezeigt ist. Das Haltegerüst 41 ist wie ein Käfig geformt und hat einen montagearmseitigen Basisendabschnitt 43, in dem eine Wellendurchführung 45 und ein Stoffzusammensetzungszuführloch 47 eingebracht ist. Des Weiteren formt der Basisendabschnitt 43 eine zylindrische Außendichtfläche 51, die formkomplementär zur Innendichtfläche 53 des Montageteils 37 geformt ist, das in Figur 5 näher dargestellt ist. Von dem montagearmseitigen Basisendabschnitt 43 erstrecken sich vier Säulen 55 in Axialrichtung A. Die Säulen 55 münden in einem montagearmfernen Basisendabschnitt 57, der ebenfalls eine zylindrische Außendichtfläche 61 formt.

Es sei angemerkt, dass die Säulen 55 sowie auch die Stützen 25 in deren axialen Verlauf ihren Querschnitt nicht ändern. Wie in Figur 1 ersichtlich ist, ist an dem montagearmfernen Basisendabschnitt 57 ein plattenförmiges Klemmteil 63 befestigt, das näher in Figur 4 dargestellt ist. An der axialen Innenseite des Klemmteils 63 ist exzentrisch zu dessen Achse eine im Wesentlichen zylindrische Einbuchtung eingebracht, in die eine (nicht dargestellte) formkomplementäre zylindrische Auswölbung des Haltegerüsts 41 eingesteckt werden kann, um eine Verdrehsicherung zwischen dem Haltegerüst 41 und dem Klemmteil 63 zu bilden.

Die Dialysezelle 7 hat eine Dialysemembran 65, die vollständig das Traggerüst 41 umgibt und an den Außenseiten der Säulen 55 sowie der Außendichtflächen 51, 61 anliegt. Um den Zellenraum 67 der Dialysezelle 7 abzudichten, wird die Membran 65 an den Außendichtflächen 51, 61 sowie den Innendichtflächen 53, 71 des Montageteils 37 bzw. des Klemmteils 63 fest eingeklemmt.

Die Dialysemembran 65 hält die Substanz, insbesondere den Wirkstoff der Stoffzusammensetzung so lange in dem Zelleninnenraum zurück, wie er an dem Molekularträger gebunden ist. Ist er von diesem freigesetzt, kann er ungehindert zusammen mit dem Substanzaufnahmefluid 73 aus der Dialysezelle 7 heraus in den Aufnahmebehälter 11 entweichen. In dem Gefäß 11 kann die Konzentration der Substanz erfasst werden, wodurch das Freisetzungsverhalten ermittelbar ist.

Beim sogenannten "reversed-dialysis"-In-vitro-Freisetzungstestverfahren wird die Substanz nicht in den Zelleninnenraum 67 gegeben, aus dem sie in ungebundenem Zustand mit dem Substanzaufnahmefluid 73 aus der Dialysezelle 7 heraus in den Aufnahmebehälter 11 migriert, sondern die Substanz wird außerhalb der Dialysezelle 7 in den Aufnahmebehälter 1 1 gegeben, so dass die Substanz sich außerhalb des Zelleninnenraums 67 von der Bindung an Molekularträger löst und ungebunden durch die Dialysemembran 65 ungehindert in das Innere der Dialysezelle 7 vordringen kann. Bei beiden Verfahren kann die Konzentration der Substanz auch im Zelleninnenraum 67 ermittelt werden, beispielsweise, indem eine Probe durch die Zuführbohrung 97 aus der Dialysezelle 7 entnommen wird.

Um eine sogenannte Aggregation (Verklumpung) der Molekularträger innerhalb des Zellinnenraums 67 zu vermeiden, ist im Zellinnenraum 67 ein Innenrührer 81 vorgesehen, der ein Innenrührorgan 83 als Exzenter zu einer Antriebswelle 85 des Antriebsstrangs 31 bildet. Das Innenrührorgan 83 umfasst einen zentralen Durchgang, in dem das Innenrührorgan 83 auf die Antriebswelle 85 aufgesteckt ist.

An den zwei exzentrischen Rührflügeln des Innenrührorgans 83 sind jeweils ein Dauermagnet 87 angeordnet, der dazu dient, einen Außenrührer 91 anzutreiben, der an einer ebenen planen Außenfläche 93 des Klemmteils 63 gehalten wird.

Die Antriebswelle 85 besteht aus einem dialysezellenseitigen Abschnitt, der von einem antriebsseitigen Abschnitt trennbar ist. Die Antriebswellenteile sind ineinander gesteckt und mit einem Sicherungsstift 95 gesichert.

Wie in Figur 1 ersichtlich ist, ist eine Zuführbohrung 97 sowohl in dem Grundendabschnitt 27 des Montagearms 17 als auch in dem Montageteil 37 sowie in dem Basisendabschnitt 43 eingebracht, um die Dialysezelle 7 mit der Stoffzusammensetzung zu befüllen oder Proben zu entnehmen. Ein Verschlusspfropfen 101 dient dazu, die Zuführöffnung zu verschließen.

Der Deckel 13 hat eine verschließbare Öffnung 19 zum Befüllen des Gefäßes 1 1 mit dem Substanzaufnahmefluid 73 und um während eines In-vitro-Freisetzungstests aus dem Behälterinneren und insbesondere aus der Dialysezelle 7 Proben entnehmen zu können. Die Öffnung 19 des Deckels 13 kann beispielsweise durch einen (nicht dargestellten) Deckeleinsatz verschließbar sein, welcher kreis- oder sichelförmig sein kann und einen einfachen manuellen Zugriff auf die Dialysezelle während eines Tests ermöglicht. Zwischen dem Deckel 13 und dem Deckeleinsatz kann, wie zwischen dem Deckel 13 und dem Gefäß 1 1, ein Dich körper, wie ein Dichtring oder eine Dichtschnur, vorgesehen sein, so dass das Gefäß 1 1 während eines In-vitro-Freisetzungstests abdichtend verschlossen ist, solange keine Probenentnahme erfolgt.

Der Außenrührer 91 umfasst zwei in Axial und Radialrichtung den Dauermagneten 87 diametral gegenüberliegenden ferromagnetischen oder dauermagnetischen Elemente 103, so dass gegenseitige Anziehkräfte zwischen den Magneten 103, 87 wirken. Diese Haltekräfte bewirken einerseits, dass der Außenrührer an der Haltefläche 93 gehalten wird. Des Weiteren bewirken die Kopplungskräfte, dass bei einer Antriebsbewegung des Innenrührers 81 der Außenrührer 91 mitgetrieben wird, so dass das Substanzaufnahmefluid 73 innerhalb des Gefäßes 11 vermengt wird. Die in der vorstehenden Beschreibung, den Figuren und den Ansprüchen offenbarten Merkmale können sowohl einzeln als auch in beliebiger Kombination für die Realisierung der Erfindung in den verschiedenen Ausgestaltungen von Bedeutung sein.

**Bezugszeichenliste**

| | | | |
|---|---|---|---|
| 1 | In-vitro-Freisetzungstestapparatur | 55 | Säulen |
| | | 63 | plattenförmiges Klemmteil |
| 3 | Aufnahmebehälter | 65 | Membran |
| 5 | Antriebseinrichtung | 67 | Zellinnenraum |
| 7 | Dialysezelle | 73 | Substanzaufnahmefluid |
| 11 | Gefäß | 81 | Innenrührer |
| 13 | Deckel | 83 | Innenrührorgan |
| 15 | O-Ring | 85 | Antriebswelle Dauermagnet |
| 17 | Montagearm | 91 | Außenrührer |
| 19 | Öffnung | 93 | plane Außenfläche |
| 21, 27 | Grundendabschnitt | 95 | Sicherungsstift |
| 23 | Bohrungen | 97 | Zuführbohrung |
| 25 | ein Paar Stützen | 101 | Verschlusspfropfen |
| 31 | Antriebstrangs | 103 | dauermagnetisches Element |
| 33 | Absatz | A | Axialrichtung |
| 35 | Tragstruktur | | |
| 37 | ringplattenförmige Montageteil | | |
| 41 | Traggerüst | | |
| 43, 57 | Basisendabschnitt | | |
| 45 | Wellendurchführung | | |
| 47 | Stoffzusammensetzungszuführloch | | |
| 51, 61 | zylindrische Außendichtfläche | | |
| 53, 71 | Innendichtfläche | | |

## Patentansprüche

1. Dialysezelle (7) für eine In-vitro-Freisetzungstestapparatur (1) zum Ermitteln eines Freisetzungsverhaltens, wie
einer Freisetzungsrate, einer Substanz einer Stoffzusammensetzung, wie eines Arzneimittels, wobei die Substanz an Molekularträger, wie Liposome, Mizellen oder Nanopartikel, gebunden ist, gegenüber einem Substanzaufnahmefluid (73), umfassend:
- einen Zelleninnenraum (67) und eine den Zelleninnenraum (67) zumindest teilweise begrenzende Dialysemembran (65), die
- die gebundene Substanz zurückhält,
- für in das Substanzaufnahmefluid (73) freigesetzte Substanz durchlässig ist und
- für das Substanzaufnahmefluid (73) zumindest teilweise durchlässig ist,
- eine Tragstruktur (35),
wobei die Dialysemembran (65) an der Tragstruktur (35) befestigt ist, welche zur festen Montage der Dialysezelle (7) an einer in Bezug zu der In-vitro-Freisetzungstestapparatur (1) ortsfesten Montageposition ausgelegt ist, wobei die Tragstruktur (35)
- ein Haltegerüst (41) und
- ein apparaturseitiges Montageteil (37) aufweist, das
- als Montageplatte (37) mit einem Außenringflansch ausgebildet ist, an dem die Tragstruktur (35) an die In-vitro-Freisetzungstestapparatur (1) lösbar befestigbar ist, und
- das Montageteil (37) gegenüber dem Haltegerüst (41) als separates Bauteil derart an dem Haltegerüst (41) lösbar befestigt ist, dass die Dialysemembran (65) zwischen dem Haltegerüst (41) und dem Montageteil (37) eingeklemmt ist, um eine den Zelleninnenraum (67) gegenüber einer Außenseite der Tragstruktur (35) geschlossen umlaufende Dichtfläche zu bilden,
**dadurch gekennzeichnet, dass**
- das Montageteil (37)
- einen Durchgang für einen in Axialrichtung (A) erstreckenden Antriebsstrang, wie eine Antriebswelle, zum Betätigen eines im Zelleninnenraum (67) anzuordnenden Innenrührer und
- eine zylindrische Montagefläche, die vorzugsweise radial nach außen weist, und eine zylindrische Dichtfläche (53) aufweist, die vorzugsweise radial nach innen auf das Haltegerüst (41) weist,
wobei das Haltegerüst (41) eine an die Dichtfläche (53) formangepasste, radial nach außen gewandte, zylindrische Dichtgegenfläche (51) umfasst,
wobei zwischen der Dichtfläche (53) und der Dichtgegenfläche (51) die Dialysemembran (65) eingeklemmt ist.

2. Dialysezelle (7) nach Anspruch 1,
**dadurch gekennzeichnet, dass**
das Haltegerüst (41)
- eine käfigartige Säulenstruktur formt und/oder
- zwei sich gegenüberliegende, vorzugsweise plattenförmige Basisendabschnitte (43, 57) sowie
- die Basisendabschnitte (43, 57) koppelnde Säulen (55) umfasst, die sich parallel einem sich in dem Zelleninnenraum (67) anzuordnenden Antriebsstrang erstrecken und/oder mit den Basisendabschnitten (43, 57) einen von der Dialysemembran (65) radial geschlossene Fensterrahmen bilden,
- wobei ausschließlich über die durch den Fensterrahmen begrenzte Fensteröffnung der Substanzdurchlass durch die Dialysemembran (65) erfolgt,
- wobei insbesondere radiale Außenseiten der Basisendabschnitte (43, 57) und der Säulen (55) in einer gemeinsamen zylindrischen Hüllebene derart liegen, dass die Dialysemembran (65) im wesentlichen spannungsarm an den Außenseiten flächig anliegt,
- wobei der apparaturnahe Basisendabschnitt (43) und das Montageteil (37) eine fluchtende Einführöffnung (47) aufweisen, über den die Stoffzusammensetzung dem Zelleninnenraum (67) zuführbar ist.

3. Dialysezelle (7) nach einem der vorstehenden Ansprüche,
**dadurch gekennzeichnet, dass**
die Tragstruktur (35) ein montageteilfernes, plattenförmiges, Klemmteil (63) zum Einklemmen der Dialysemembran (65) an ein Haltegerüst der Tragstruktur (35) umfasst,
wobei das Klemmteil (63) eine zylindrische Dichtfläche (71) aufweist, die radial nach innen auf das Haltegerüst (41) weist, das eine an die Dichtfläche (71) formangepasste, radial nach außen gewandte, zylindrische Dichtgegenfläche (61) formt,
wobei zwischen der Dichtfläche (71) und der Dichtgegenfläche (61) die Dialysemembran (65) eingeklemmt ist,
wobei das Klemmteil (63) eine von dem Zelleninnenraum (67) abgewandte insbesondere plane Tragfläche (93) formt, an der ein Außenrührer (91) gehalten ist.

4. Dialysezelle (7) nach einem der vorstehenden Ansprüche,
**dadurch gekennzeichnet, dass**
- die Dialysemembran (65) die Form eines flexiblen, im montierten Zustand in dessen Längsrichtung konstant-hohlzylinderförmigen Schlauchs aufweist und/oder
- die Tragstruktur (35) derart mantelförmig umgibt, dass durch die Dialysemembran (65) gebildete Zellinnenraumgrenzwände sich parallel zu einer Längsachse der Tragstruktur (35), zu einem in den Zelleninnenraum (67) ragenden Antriebsstrang (31), wie einer Antriebswelle (85), zum Betätigen eines im Zelleninnenraum (67) anzuordnenden Innenrührers (81), erstrecken.

5. Dialysezelle (7) nach einem der vorstehenden Ansprüche,
**dadurch gekennzeichnet, dass**
im Zelleninnenraum (67) ein Innenrührer (81) mit einem im Zelleninnenraum (67) vollständig liegendem Innenrührorgan (83) und gegebenenfalls einer mit dem Innenrührorgan (83) direkt gekoppelten Antriebswelle (85) angeordnet ist, die sich von dem Innenrührorgan (83) aus der Dialysezelle (7) heraus dichtend erstreckt,
wobei das Innenrührorgan (83) dauermagnetisch ist und/oder ein Steckteil zum Halten eines Dauermagneten, insbesondere eines Dauermagnetenpaares, am Ende der Antriebswelle (85) vorzugsweise abnehmbar, aufgesteckt ist,
wobei sich die direkte Antriebswelle (85) für das Innenrührorgan (83) durch eine Lageröffnung (45) sowohl in dem Basisendabschnitt (43) der Tragstruktur (35) als auch in dem Montageteil (37) der Tragstruktur (35) hindurch erstreckt und/oder eine Dichtung zwischen der Antriebswelle (85) und der Tragstruktur (35) vorgesehen ist.

6. Dialysezelle (7) nach einem der vorstehenden Ansprüche,
**dadurch gekennzeichnet, dass**
an einem montageteilfernem Abschnitt der Tragstruktur (35) außenseitig ein Außenrührer (91) angeordnet ist, der mit einem in dem Zelleninnenraum (67) angeordneten Innenrührer (81) derart berührungslos gekoppelt ist, dass ein direkter Antrieb für einen Rührer (81, 91), insbesondere den Innenrührer (81), auch den anderen Rührer (91, 81) antreibend betätigt,
wobei ein Außenrührorgan des Außenrührers (91) dauermagnetisch ist oder zumindest derart ferromagnetisch reagiert, dass das Außenrührorgan unter dem Einfluss magnetischer Anziehungskräfte einem getriebenen Innenrührorgan (83) des Innenrührers (81) folgt und/oder an der Tragstruktur (35) gehalten wird.

7. Verwendung einer nach einem der vorstehenden Ansprüche ausgebildeten Dialysezelle (7) für eine In-vitro-Freisetzungstestapparatur (1) insbesondere nach Anspruch 8 oder 9.

8. In-vitro-Freisetzungstestapparatur (1) zum Ermitteln eines Freisetzungsverhaltens, wie einer Freisetzungsrate, einer Substanz einer Stoffzusammensetzung, wie eines Arzneimittels, welche Substanz an Molekularträger, wie Liposome, Mizellen oder Nanopartikel, gebunden ist, gegenüber einem Substanzaufnahmefluid (73), umfassend
eine nach einem der vorstehenden Ansprüche 1 bis 6 ausgebildete Dialysezelle (7) und
einen die Dialysezelle (7) zumindest teilweise umgebenden Umgebungsraum zum Aufnehmen des Substanzaufnahmefluids (73).

9. In-vitro-Freisetzungstestapparatur (1) nach Anspruch 8,
mit einem Aufnahmebehälter (3) zum Begrenzen des Umgebungsraums, von dessen Wandung sich ein Montagearm (17) in das Behälterinnere erstreckt, der einen endseitigen Montageflansch aufweist, an dem die Tragstruktur (35) der Dialysezelle (7) befestigt ist,
wobei der Montagearm (17) lösbar an der Wandung befestigt ist und/oder eine käfigartige Säulenstruktur formt und/oder zwei sich gegenüberliegende, plattenförmige Grundendabschnitte sowie die Grundendabschnitte koppelnde Stützen umfasst, die sich parallel zu einem in dem Behälterinnenraum anzuordnenden Antriebsstrang erstrecken,
wobei ein Antriebsstrang (31), vorzugsweise eine Antriebswelle (85), für einen in dem Zelleninnenraum (67) angeordneten Innenrührer (81) sich durch die Wandung erstreckt, insbesondere an der Wandung gelagert ist, und/oder zweigeteilt ist,
wobei sich ein zellenseitiges Strangteil von dem Zelleninnraum (67) in den Aufnahmebehälter (3) und ein zellenfernes Strangteil von dem Behälterinneren zur Außenseite des Aufnahmebehälters (3) erstrecken.

## Claims

1. Dialysis cell (7) for an *in-vitro* release test apparatus (1) for determining a release behaviour such as a release rate of a substance of a material composition such as a drug, wherein the substance is bound to molecular carriers such as liposomes, micelles or nanoparticles with respect to a substance uptake fluid (73), comprising:
- a cell interior (67) and a dialysis membrane (65) at least partially delimiting the cell interior (67), which
- holds the bound substance back,
- is permeable for substance released into the substance uptake fluid (73) and
- is at least partly permeable for the substance uptake fluid (73)
- a support structure (35), wherein the dialysis membrane (65) is attached to the support structure (35), which is designed for fixed mounting of the dialysis cell (7) at a mounting position that is immobile relative to the *in-vitro* release test apparatus (1), wherein the support structure (35) includes
- a retaining enclosure (41) and
- a mounting element (37) which is designed for attachment to the apparatus, which
- is embodied as a mounting plate (37) with an outer ring flange by which the support structure (35) can be fastened detachably to the *in-vitro* release test apparatus (1), and
- the mounting element (37) is fastened to the retaining enclosure (41) detachably as a separate component with regard to the retaining enclosure (41) in such manner that the dialysis membrane (65) is clamped between the retaining enclosure (41) and the mounting element (37), thereby forming a sealing surface which completely surrounds the cell interior (67), closing it off from an outside area of the support structure (35),
**characterized in that**
- the mounting element (37)
- has a passage for a drivetrain extending in axial direction (A), such as a driveshaft for operating an internal agitator unit which is to be disposed in the cell interior (67), and
- a cylindrical mounting surface, which preferably faces radially outwardly, and a cylindrical sealing surface (53) which preferably faces radially inwardly towards the retaining enclosure (41),
wherein the retaining enclosure (41) comprises a cylindrical mating sealing surface (51) with a shape adapted to the sealing surface (53) and facing radially outwards,
wherein the dialysis membrane (65) is clamped between the sealing surface (53) and the mating sealing surface (51).

2. Dialysis cell (7) according to Claim 1,
**characterized in that** the retaining enclosure (41)
- forms a cage-like column structure, and/or
- comprises two oppositely positioned, preferably plate- like base end sections (43, 57) and
- columns (55) that connect the base end sections (43, 57), which columns extend parallel to a drivetrain which is to be disposed in the cell interior (67) and/or together with the base end sections (43, 57) form a window frame which is closed radially by the dialysis membrane (65),
- wherein the substance is permitted to pass through the dialysis membrane (65) solely via the window opening which is delimited by the window frame,
- wherein in particular radial outer sides of the base end sections (43, 57) and of the columns (55) lie in a common cylindrical enveloping plane in such manner that the dialysis membrane (65) lies flat against the outer sides substantially under low tension,
- wherein the base end section (43) closest to the apparatus and the mounting element (37) have an aligned infeed opening (47) through which the material composition can be introduced into the cell interior (67).

3. Dialysis cell (7) according to any one of the preceding claims,
**characterized in that**
the support structure (35) comprises a plate-like clamping element (63) at an opposite end to the mounting element for clamping the dialysis membrane (65) to a retaining enclosure of the support structure (35),
wherein the clamping element (63) has a cylindrical sealing surface (71) which faces radially inwardly towards the retaining enclosure (41), which forms a cylindrical mating sealing surface (61) facing radially outwardly and adapted to the shape of the sealing surface (71),
wherein the dialysis membrane (65) is clamped between the sealing surface (71) and the mating sealing surface (61),
wherein the clamping element (63) forms a bearing surface (93) which is in particular flat and facing away from the cell interior (67), on which an external agitator unit (91) is retained.

4. Dialysis cell (7) according to any one of the preceding claims,
**characterized in that**
- the dialysis membrane (65) has the form of a flexible hose which in the mounted state has a constant hollow-cylindrical form in the longitudinal direction thereof, and/or
- surrounds the support structure (35) in the manner of a mantle such that boundary walls of the cell interior formed by the dialysis membrane (65) extend parallel to a lengthwise axis of the support structure (35) and parallel to a drivetrain (31) that protrudes into the cell interior (67), such as a driveshaft (85) for operating an internal agitator unit (81) which is to be disposed in the cell interior (67).

5. Dialysis cell (7) according to any one of the preceding claims,
**characterized in that**
an internal agitator unit (81) is arranged in the cell interior (67) and has an internal stirrer (83) which is located entirely inside the cell interior (67) and optionally a driveshaft (85) which is coupled directly with the internal agitator unit (83) and extends in sealing manner from the internal agitator unit (83) and out of the dialysis cell (7),
wherein the internal stirrer (83) is permanently magnetised and/or a preferably removable plug-in element is inserted at the end of the driveshaft (85) to hold a permanent magnet, particularly a permanent magnet pair,
wherein the direct driveshaft (85) for the internal stirrer (83) passes through a bearing opening (45) in both the base end section (43) of the support structure (35) and in the mounting element (37) of the support structure (35) and/or a seal is provided between the driveshaft (85) and the support structure (35).

6. Dialysis cell (7) according to any one of the preceding claims,
**characterized in that**
an external agitator unit (91) is arranged externally on a section of the support structure (35) which is remote from the mounting element, and is coupled contactlessly with an internal agitator unit (81) which is arranged in the cell interior (67) in such manner that a direct drive for one agitator unit (81, 91), particularly for the internal agitator unit (81), also operates the other agitator unit (91, 81) in driving manner,
wherein an external stirrer of the external agitator unit (91) is permanently magnetised or at least reacts ferromagnetically such that the external stirrer follows a driven internal stirrer (83) of the internal agitator unit (81) and/or is held on the support structure (35) under the influence of magnetic attracting forces.

7. Use of a dialysis cell (7) embodied according to any one of the preceding claims for an *in-vitro* release test apparatus (1), in particular according to Claim 8 or 9.

8. *In-vitro* release test apparatus (1) for determining a release behaviour such as a release rate of a substance of a material composition such as a drug, which substance is bound to molecular carriers such as liposomes, micelles or nanoparticles with respect to a substance uptake fluid (73), comprising
a dialysis cell (7) embodied according to any one of the preceding Claims 1 to 6 and
an ambient space at least partially surrounding the dialysis cell (7), preferably completely, for accommodating the substance uptake fluid (73).

9. *In-vitro* release test apparatus (1) according to Claim 8,
with a receptacle (3) for delimiting the ambient space, from whose wall a mounting arm (17) extends into the interior of the receptacle and has a mounting flange on the end thereof, to which the support structure (35) of the dialysis cell (7) is fastened,
wherein the mounting arm (17) is fastened releasably to the wall and/or forms a cage-like columnar structure and/or comprises two oppositely positioned, preferably plate-like base end sections and the supports that couple the base end sections, which extend parallel to a drivetrain that is to be arranged in the receptacle interior,
wherein a drivetrain (31), preferably a driveshaft (85) for an internal agitator unit (81) arranged in the cell interior (67) extends through the wall, in particular is supported on the wall, and/or is divided into two parts,
wherein a drivetrain part on the cell side extends from the cell interior (67) into the receptacle (3), and a drivetrain part distant from the cell extends from the receptacle interior to the outside of the receptacle (3).

## Revendications

1. Cellule de dialyse (7) pour appareil de test de libération in vitro (1) pour la détermination d'un comportement de libération comme une vitesse de libération, d'une substance d'une composition de substances, comme un médicament, la substance étant liée à des supports moléculaires comme des liposomes, des micelles ou des nanoparticules, par rapport à un fluide récepteur de substance (73), comprenant :
- un espace intérieur de cellule (67) et une membrane de dialyse (65) limitant du moins partiellement l'espace intérieur de cellule (67) et qui
- retient la substance liée,
- est perméable à la substance libérée dans le fluide récepteur de substance (73) et
- est perméable du moins partiellement au fluide récepteur de substance (73),
- une structure porteuse (35), la membrane de dialyse (65) étant fixée à la structure porteuse (35) qui, pour un montage fixe de la cellule de dialyse (7), est posée à une position de montage fixe par rapport à l'appareil de test de libération in vitro (1), la structure porteuse (35) présentant
- une structure de retenue (41) et
- une pièce de montage (37) située au niveau de l'appareil et qui
- est réalisée sous forme d'une plaque de montage (37) dotée d'une bride annulaire extérieure au niveau de laquelle la structure porteuse (35) peut être fixée de manière dissociable à l'appareil de test de libération in vitro (1), et
- la pièce de montage (37) étant fixée à la structure de retenue (41) de manière dissociable par rapport à la structure de retenue (41) sous forme d'une pièce séparée de manière à ce que la membrane de dialyse (65) soit serrée entre la structure de retenue (41) et la pièce de montage (37) afin de constituer une surface d'étanchéité entourant l'espace intérieur de cellule (67) de manière à le fermer par rapport à une face extérieure de la structure porteuse (35),
**caractérisée en ce que**
- la pièce de montage (37)
- présente un passage pour une chaîne cinématique s'étendant dans le sens axial (A), comme un arbre d'entraînement, pour actionner un mélangeur intérieur à disposer dans l'espace intérieur de cellule (67) et
- une surface de montage cylindrique qui est de préférence tournée radialement vers l'extérieur, et présente une surface d'étanchéité cylindrique (53) qui est de préférence tournée radialement vers l'intérieur en direction de la structure de retenue (41),
la structure de retenue (41) comprenant une contre-surface d'étanchéité cylindrique (51) de forme adaptée à la surface d'étanchéité (53) et tournée radialement vers l'extérieur,
la membrane de dialyse (65) étant serrée entre la surface d'étanchéité (53) et la contre-surface d'étanchéité (51).

2. Cellule de dialyse (7) selon la revendication 1,
**caractérisée en ce que**
la structure de retenue (41)
- forme une structure en colonne ressemblant à une cage et/ou
- comprend deux sections terminales de base opposées de préférence en forme de plaques (43,57) et
- les sections terminales de base (43,57) comprennent des colonnes de couplage (55) qui s'étendent parallèlement à une chaîne cinématique à disposer dans l'espace intérieur de cellule (67) et/ou constituent avec les sections terminales de base (43,57) un cadre de fenêtre fermé radialement par la membrane de dialyse (65),
- le passage de substance à travers la membrane de dialyse (65) ayant exclusivement lieu par l'ouverture de fenêtre limitée par le cadre de fenêtres,
- les faces extérieures radiales des sections terminales de base (43,57) et des colonnes (55) se trouvant en particulier dans un plan d'enveloppement cylindrique commun de manière à ce que la membrane de dialyse (65) repose sensiblement en surface et sans tension au niveau des faces extérieures,
- la section terminale de base (43) proche de l'appareil et la pièce de montage (37) présentant une ouverture d'introduction alignée (47) par laquelle la composition de substances peut être acheminée vers l'espace intérieur de cellule (67).

3. Cellule de dialyse (7) selon une des revendications précédentes,
**caractérisée en ce que**
la structure porteuse (35) comprend une pièce de serrage (63) en forme de plaque éloignée de la pièce de montage pour serrer la membrane de dialyse (65) contre une structure de retenue de la structure porteuse (35),
la pièce de serrage (63) présentant une surface d'étanchéité cylindrique (71) qui est tournée radialement vers l'intérieur en direction de la structure de retenue (41) qui forme une contre-surface d'étanchéité (61) cylindrique adaptée à la forme de la surface d'étanchéité (71) et tournée radialement vers l'extérieur,
la membrane de dialyse (65) étant serrée entre la surface d'étanchéité (71) et la contre-surface d'étanchéité (61),
la pièce de serrage (63) formant une surface porteuse (93) en particulier plane, détournée de l'espace intérieur de cellule (67) et au niveau de laquelle un mélangeur extérieur (91) est maintenu.

4. Cellule de dialyse (7) selon une des revendications précédentes,
**caractérisée en ce que**
- la membrane de dialyse (65) présente la forme d'un tuyau souple, en état monté de forme constamment cylindrique creuse dans son sens longitudinal et/ou
- entoure la structure porteuse (35) en forme de manteau de manière à ce que les parois de limitation de l'espace intérieur de cellule formées par la membrane de dialyse (65) s'étendent parallèlement à un axe longitudinal de la structure porteuse (35) pour former une chaîne cinématique (31) dépassant dans l'espace intérieur de cellule (67), comme un arbre d'entraînement (85), pour actionner un mélangeur intérieur (81) à disposer dans l'espace intérieur de cellule (67).

5. Cellule de dialyse (7) selon une des revendications précédentes,
**caractérisée en ce que**,
dans l'espace intérieur de cellule (67) est disposé un mélangeur intérieur (81) doté d'un organe de mélange intérieur (83) se trouvant entièrement dans l'espace intérieur de cellule (67) et, le cas échéant, un arbre d'entraînement (85) couplé directement à l'organe de mélange intérieur (83) et qui s'étend de manière étanche en partant de l'organe de mélange intérieur (83) hors de la cellule de dialyse (7),
l'organe de mélange intérieur (83) étant durablement magnétique et/ou une pièce de fichage servant à retenir un aimant permanent, en particulier une paire d'aimants permanents, étant fichée à l'extrémité de l'arbre d'entraînement (85), de préférence de manière amovible,
l'arbre d'entraînement direct (85) pour l'organe de mélange intérieur (83) s'étendant à travers une ouverture de palier (45) aussi bien à travers la section terminale de base (43) de la structure porteuse (35) que dans la pièce de montage (37) de la structure porteuse (35) et/ou un joint étant prévu entre l'arbre d'entraînement (85) et la structure porteuse (35).

6. Cellule de dialyse (7) selon une des revendications précédentes,
**caractérisée en ce que**,
au niveau d'une section éloignée de la pièce de montage de la structure porteuse (35), à l'extérieur, est disposé un mélangeur extérieur (91) qui est couplé sans contact à un mélangeur intérieur (81) disposé dans l'espace intérieur de cellule (67) de manière à ce qu'un entraînement direct pour un mélangeur (81,91), en particulier le mélangeur intérieur (81), actionne en l'entraînant également l'autre mélangeur (91,81),
un organe de mélange extérieur du mélangeur extérieur (91) étant durablement magnétique ou au moins réagissant au niveau ferromagnétique de manière à ce que l'organe de mélange extérieur, sous l'influence de forces d'attraction magnétiques, suive un organe de mélange intérieur entraîné (83) du mélange intérieur (81) et/ou soit maintenu au niveau de la structure porteuse (35).

7. Utilisation d'une cellule de dialyse (7) réalisée selon une des revendications précédentes pour un appareil de test de libération in vitro (1), en particulier selon la revendication 8 ou 9.

8. Appareil de test de libération in vitro (1) pour la détermination d'un comportement de libération, comme une vitesse de libération, d'une substance d'une composition de substances, comme un médicament, la substance étant liée à des supports moléculaires comme des liposomes, des micelles ou des nanoparticules, par rapport à un fluide récepteur de substance (73), comprenant :
une cellule de dialyse (7) réalisée selon une des revendications précédentes 1 à 6 et
un espace environnant entourant au moins partiellement la cellule de dialyse (7) et destiné à recevoir le fluide récepteur de substance (73).

9. Appareil de test de libération in vitro (1) selon la revendication,
comportant un réceptacle (3) pour limiter l'espace environnant, réceptacle depuis la paroi duquel un bras de montage (17) s'étendant dans l'intérieur du réceptacle, lequel bras de montage présente une bride montage terminale à laquelle la structure porteuse (35) de la cellule de dialyse (7) est fixée,
le bras de montage (17) étant fixé de manière dissociable à la paroi et/ou formant une structure en colonne ressemblant à une cage et/ou comprenant deux supports opposés en forme de plaques couplant les sections terminales de base et qui s'étendent parallèlement à une chaîne cinématique à disposer dans l'espace intérieur du récipient,
une chaîne cinématique (31), de préférence un arbre d'entraînement (85) pour un mélangeur intérieur (81) disposé dans l'espace intérieur de cellule (67), s'étendant à travers la paroi, en particulier en s'appuyant à la paroi, et/ou étant divisé en deux,
une pièce de chaîne située du côté de la cellule s'étendant depuis l'espace intérieur de cellule (67) dans le réceptacle (3) et une pièce de chaîne éloignée de la cellule s'étendant depuis l'intérieur du récipient jusqu'à la face extérieure du réceptacle (3).
